# EUROPEAN PATENT APPLICATION

(11) **EP 2 298 390 A1**
(43) Date of publication of application: **23.03.2011**
(21) Application number: 09011031.3
(22) Date of filing: 28.08.2009
(51) Int. Cl.: A61M 5/178

(54) **Medical delivery device, housing cap for a medical delivery device and drive mechanism for a medical delivery device**

(71) Applicant: Sanofi-Aventis Deutschland GmbH, 65929 Frankfurt am Main (DE)
(72) Inventor: Hamm, German F., 65926 Frankfurt am Main (DE)

(57) **Abstract**

A medical delivery device (10, 20, 40) comprising a housing (11, 21, 44, 46), a drive mechanism (A) disposed within the housing (11, 21, 44, 46) for expelling a medicament from a medicament reservoir (29), and an operating device (D) functionally coupled to the drive mechanism (A) for controlling the drive mechanism (M) when operated by a user, wherein the housing (11, 21, 44, 46) is formed by a shell made of a ceramic material, a housing cap (12) for a portable medical delivery device (10, 20, 40) at least partly made of a ceramic material, and a drive mechanism (A) for a medical delivery device (10, 20, 40), comprising a component made of a ceramic material. Further, it is provided a method of forming a medical delivery device (10, 20, 40), wherein ceramic material is used for a housing part of the medical delivery device (11, 20, 40) and/or for inner mechanical parts of the medical delivery device (10, 20, 40).

## Description

Medical delivery device, housing cap for a medical delivery device and drive mechanism for a medical delivery device

The invention relates to a medical delivery device, a housing cap for a medical delivery device and a drive mechanism for a medical delivery device. The invention also relates to the use of ceramic material in a drive mechanism for such a medical delivery device and to a method of forming a medicament delivery device.

Medical delivery devices particularly portable medical delivery devices are known for self administration of a medicament by patients. Delivery devices are normally made of plastic material in order to reduce the weight of the device and in order to be able to produce such devices most efficiently. However, by using plastic for moving parts of delivery devices, the functionality and the accuracy of moving parts is limited or the production costs increase disproportionately to achieving an increase in functionality or accuracy. Further, a good looking outer appearance and a precious design can be required by users of delivery devices.

It is an object of the invention to provide a medical delivery device which is improved in its technical stability and technical function.

It is a further object of the invention to provide a medical delivery device which provides a good looking appearance and, in particular, does not give the impression of a medical instrument.

It is also an object of the invention to provide a medical delivery device by which the surface properties of the components of a medical delivery device can be improved such that the possibility of a damage of the outer surfaces, which negatively affects the optical appearance of the medical delivery device, is reduced.

It is a further object of the invention to provide a medical delivery device which is also more reliable in use and more robust against environmental impact. In this regard, it is desirable that parts of the delivery device are resistant against alcohol and other detergents.

According to the invention, it is not only desired that the outer surfaces of the delivery device are robust and of a precious appearance, but it is also preferred to make the inner parts of the delivery device robust. In particular, the inner parts that are movable against each other should be both strong and smooth in order to be operable by a low expenditure of energy.

These objects are solved by the features of the independent claims. Further embodiments of the invention are described in the subclaims which refer back thereto. According to the invention, a medical delivery device is provided, comprising: a housing, a drive mechanism disposed within the housing for expelling medicament from a medicament reservoir, and an operating device functionally coupled to the drive mechanism for controlling the drive mechanism when operated by a user, wherein the housing is formed by a shell made of a ceramic material.

According to the invention, also provided a housing part for a medical delivery device, the delivery device comprising a housing, a drive mechanism disposed within the housing for expelling medicament from a medicament reservoir, and an operating device disposed on the housing to be operated by a user for controlling the drive mechanism, wherein the housing-part is formed as a cap removable attachable to housing, wherein the second housing part is to be removed when expelling medicament from the medicament reservoir, and wherein the second housing part is formed of a first ceramic material.

According to the invention, also a drive mechanism for a medical delivery device is provided, comprising at least one component which is rotatable or longitudinally movable with regard to the housing of the medical delivery device and which is made of a ceramic material.

Moreover, according to the invention, also a medical delivery is provided comprising a drive mechanism according to the invention as described above.

Further, according to the invention a method of forming a medical delivery device is provided, wherein a ceramic material is used for a housing part of the medical delivery device and/or for inner mechanical parts of the medical delivery device.

The invention can be used in various different kinds of medical delivery devices. The term "medicament delivery device" according to instant invention shall mean a single-dose or multi-dose or pre-set dose or pre-defined dose, disposable or re-useable device designed to dispense a user selectable or pre-defined dose of a medicinal product, preferably multiple pre-defined doses, e.g. insulin, growth hormones, low molecular weight heparins, and their analogues and/or derivatives etc. Said device may be of any shape, e.g. compact or pen-type.

Dose delivery may be provided through a mechanical (optionally manual) drive mechanism or electrical drive mechanism or electro-mechanical mechanism or stored energy drive mechanism, such as a spring, etc. Dose selection may be provided through a manual mechanism or electro-mechanical mechanism or electronic mechanism. In particular, the term "medicament delivery device" shall mean a disposable needle-based pen-type device providing multiple pre-defined doses having mechanical and manual dose delivery and an operating device, which is designed for use by persons without formal medical training such as patients. Preferably, the medicament delivery device is of the injector-type.

An example for a medicament delivery device according to the invention is an insulin pen, which comprise an elongate outer housing and a cartridge located within the housing containing the medicament. The insulin pen typically comprises a drive mechanism for driving a piston located in the cartridge for expelling medicament when the user is actuating an operating mechanism of the insulin pen.

The term "housing" according to instant invention shall preferably mean any exterior housing ("main housing", "body", "shell") or interior housing ("insert', "inner body"). The housing can consist of two ore more housing parts that can be connected and disconnected with each other. For example, one part of the housing can be a cap. The housing can comprise one or more helical threads. The housing may be designed to enable the safe, correct, and comfortable handling of the medicament delivery device or any of its mechanism. Usually, it is designed to house, fix, protect, guide, and/or engage with any of the inner components of the drug delivery device (e.g., the drive mechanism, cartridge, plunger, piston rod) by limiting the exposure to contaminants, such as liquid, dust, dirt etc. In general, the housing may be unitary or a multipart component of tubular or non-tubular shape. Usually, the exterior housing serves to house a cartridge, which may be replaceable or non-replaceable, from which a number of doses of a medicinal product may by dispensed.

At least one part of the housing is made of ceramic material, and the ceramic material can be highly polished on its outer surface. According to an embodiment of the invention, the housing is formed such that the first ceramic material is extended up to the outer surface of the housing. Optionally, the ceramic part of the housing is partly coated with an additional layer which can consist of a metal or a plastic material.

The medicine delivery device an comprise a housing that is formed such that it comprise a first connection part on which a medicament cartridge can be attached, wherein the housing supports the drive mechanism and the operating device, wherein the drive mechanism can act on the medicament cartridge for expelling the medicament from the cartridge.

The housing can comprise two or more separate housing parts. For example, one part can be designed in the form of a housing, wherein another part of the housing can be designed as a removable cap. According to a preferred embodiment, the delivery device comprises a cap removably attachable to the housing for protecting a distal end of the housing, wherein in the present application the "distal end" of the device or a component of the device shall mean the end, which is closest to the dispensing end of the device. The cap can be polished on its outer surface. In one embodiment, the shell of the cap comprises an outer surface of the housing, wherein the outer surface is at least partly polished. Further, the outer surface of the cap can be at least partly coated with a layer of plastic material or a layer of metal. The cap can be at least partly coated with a layer of a ceramic material. According to an embodiment of the invention, the material of the additional ceramic layer of the cap is different from the material of the inner part of the cap made from a ceramic material.

Further, according to an embodiment of the invention, the housing comprise a ceramic part having a friction surface that is in friction contact with parts of the drive mechanism and/or with parts of the operation mechanism, wherein the friction surface can be highly polished such that it has a low friction coefficient. Moreover, the ceramic part can at least partly be coated with a layer of a second ceramic material which may be different to the ceramic material of the ceramic part. The second ceramic material can be highly polished.

The drive mechanism comprises at least one movable component which is rotatable or longitudinally movable with regard to the housing. The term "drive mechanism" according to instant invention shall mean any module or component or set of components designed to exert force to components such as a piston that are disposed in a medicament container such as a cartridge in order to expel medicament. According to an embodiment of the invention, the movable component of the drive mechanism is made of a ceramic material. The movable component made of ceramic material can be a piston rod.

According to a preferred embodiment, the piston rod is coated with ceramic material or made of ceramic material. The term "piston rod" according to instant invention shall mean a component adapted to operate through/within the housing, designed to translate axial movement through/within the drug delivery device, for the purpose of expelling medicament. Said piston rod may be a simple rod, a lead-screw, a rack and pinion system, a worm gear system, or the like. It may further be of a unitary or multicomponent construction. The term "piston rod" shall further mean a component having a circular or non-circular cross-section.

Further, according to an embodiment, the movable component of the drive mechanism is a drive sleeve which is coated with ceramic material or made of ceramic material. According to a preferred embodiment, an inner surface and/or an outer surface of the drive sleeve which is co-acting with the piston rod is made of ceramic material or at least partly coated of a ceramic material. The term "drive sleeve" according to instant invention shall mean any essentially tubular component which is designed to transfer a movement into a movement of the piston rod. The drive sleeve is preferably of essentially circular cross-section and is further releasably connected to the operating device. In a preferred embodiment the drive sleeve is engaged with the piston rod. The engagement can be a threaded engagement.

According to the invention, the drive mechanism and/or the operating device comprise a first component that is moving with regard to the housing when the drive mechanism is actuated and a second component that is stationary with regard to the housing when the drive mechanism is actuated, wherein at least one second component is made of ceramic material or coated with ceramic material.

The term "operating device" according to instant invention shall mean any module or any component or set of components designed to be operated by the user in order to control the drive mechanism. When controlling the drive mechanism, force it transmitted to the drive mechanism. The force can be generated manually or by means of a motor. In a preferred embodiment, the operating device comprises actuating keys. In another preferred embodiment, the operating device comprises a dose dosing sleeve. The operating device can comprise components having grip surfaces to enable the user to actuate the device. In yet another preferred embodiment of instant invention the operating device is provided with a contact surface to allow the user to dispense a selected dose of medicament.

According to an embodiment of the invention, the operating device can comprise components that do not rotate with respect to the housing but is allowed to move longitudinally with respect to the housing to transmit force from the user to the drive sleeve. Additionally or alternatively, the operating device can comprise components which rotate when the user is actuating the medicament delivery device, for example when the user is setting a dose.

According to an embodiment, the operating device comprises a dosing sleeve formed of ceramic material or coated with a ceramic material.

According to the invention, different ceramic materials can be used in a medicament delivery device. For example, when a medical delivery device in accordance with the invention comprise multiple components of ceramic material, one components, for example the drive sleeve, can be formed of a first ceramic material, wherein another component, for example the housing, is formed of a second ceramic material, which is different to the first ceramic material of the drive sleeve. As another example, the housing ca can be formed of another ceramic material as the housing.

According to the invention, the parts made of ceramic material can be formed in a ceramic injection moulding (CIM) process with following refining processes. However, the invention is not restricted to a CIM process. Rather, any suitable other process can be used to form a ceramic component for use in a medical delivery device according to the invention.

An advantage of the invention is that, since ceramic material is used for components of the medical delivery device and in particular for its housing components, the device can, for example, be provided with housing components that can both easily be dyed and labelled and that also provide a high resistance against scratching and surface damage. As another advantage of the invention, because of the use of ceramic components, parts of the medical device are made biocompatible because ceramic parts can have a low toxicity and therefore a device having a high compatibleness, in comparison to a device using synthetic materials, is achieved.

In addition, parts, for example housing parts, can be provided that give a high value touch feeling when touched by a user. Housing parts can provide a cool metallic character touch feeling. This is, amongst other reasons, due to the relatively high specific weight of some ceramic materials.

Yet another advantage of the invention is that the ceramic parts and ceramic surfaces have, when they are disposed in a medical device or in a drive mechanism and when they are correspondingly treated, for example when they are high polished, provide only a low friction with regard to other components and surfaces of the device or drive mechanism when the ceramic parts and the other components are lying against each and perform movement against each other. Further, as an advantage, ceramic parts can have a low grade of wasting and erosion. Therefore, smooth and robust running of the mechanical parts when the medical device is used can be achieved.

The parts formed of ceramic material do not need to be formed by a raw material of hundred percent ceramic but can also comprise a fraction of other source materials, such as plastic.

The ceramic parts used in a medicine delivery device in accordance with the invention can be formed using a ceramic injection moulding (CIM) process. In such a process, typically a mixture of plastic- and ceramic granules are fluidised in a conventional ceramic injection moulding apparatus. Then, the melt is injected into a injection moulding tool. Then the melt is solidifying and the casting piece ("green body") is formed. Then, as a debinding process and to remove the plastic from the ceramic, the green body is baked. Alternatively, another debinding process will be carried out, for example a chemical treatment. Then, in a sintering step, the resulting ceramic part is baked. Finally, the ceramic part will be cooled and finished and or polished. In the finishing, other additional steps, such as a milling step can be conducted.

Herein, the term "distal" according to instant invention shall mean a direction or position which is directed or positioned, respectively, to the dispensing end of the device or of a component of the device. The term "proximal" according to instant invention shall mean a direction or position which is directed away from the dispensing end of the device or of a component of the device or is positioned, respectively, to at an end of the device or of a component of the device lying opposite to a distal position.

The invention will now be described, by way of example only, with reference to the accompanying-drawings in which:
Figure 1 shows a perspective view of a medical delivery device according to a first embodiment of the invention;
Figure 2 shows a sectional view of a medical delivery device according to a second embodiment of the invention, wherein the components of the drive mechanism and the operating device are in a first position or cartridge full position;
Figure 3 shows a sectional view of the medical delivery device according to the second embodiment, wherein the components of the drive mechanism and the operating device are in a second position or dose setting position; and
Figure 4 shows a sectional view of a medicament delivery device according to a third embodiment of the invention;

In the following detailed description, components with similar function which are common to the embodiments described in connection with the Figs. are denoted by the same reference numerals, wherein repetition in describing such same components is avoided as possible.

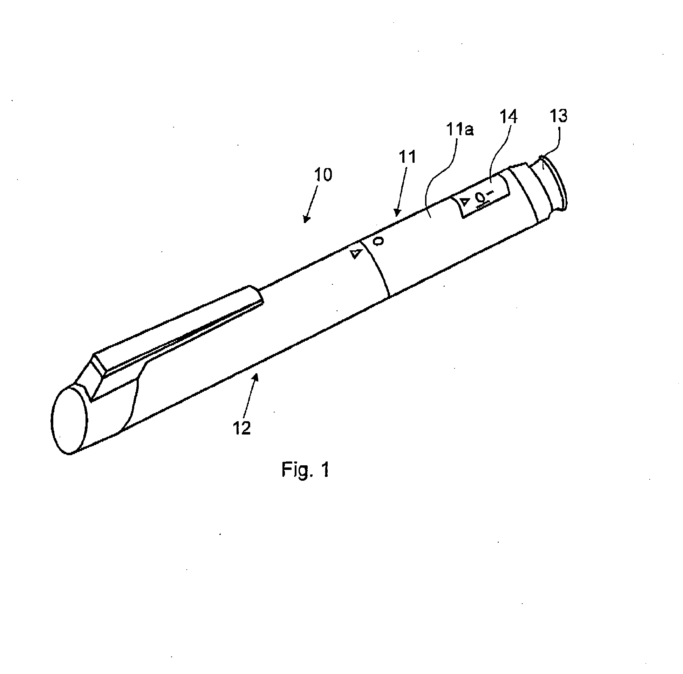

Referring to figure 1, a medical delivery device 10 according to a first embodiment of the invention is shown, which has the design of a pen, for example an insulin pen.

The medical delivery device 10 comprises a housing 11 including a main housing 11 a and a front housing. In the front housing a medicament cartridge (not shown) and a drive mechanism (not shown) can be disposed. The medicament delivery device also comprises a housing cap 12 for covering the front housing. In figure 1 the front part of the housing 11 is not visible since it is covered by the housing cap 12. Further, the medicament delivery device 10 includes an operating device within the main housing 11a. When the medicament delivery device 10 is used, the housing cap 12 is to be removed in order to expel a medicament. In order to set of dose or to administer the dose of a medicament, the user actuates the medicament delivery device 10 by means of the operating device 10. For example, if the user wishes to expel a medicament from the cartridge, he presses the operating button 13 which is a component of the operating device. The operating device is functionally coupled with the drive mechanism. The drive mechanism drives a piston, which is movable within the medicament cartridge (not shown) such that the medicament is expelled from the cartridge.

According to the invention, the main housing 11 a can be made of a ceramic material. Further, the housing cap 12 can be made of a ceramic material which in particular can be the same as the ceramic material of the main housing 11a or, alternatively, which is different to this ceramic material. Also, the operating button 13 can be made of a ceramic material.

By using a ceramic material for forming a part of the housing 11 and/or the cap 12 and/or parts of the operating device, the delivery device 10 can be made very robust and resistant against environmental impact and against detergents. Further, the scratching resistance of the corresponding surfaces is increased and give the impression of a high-value appearance.

The operating button 13 shown in figure 1 comprise a rearward circular surface and a annular cone formed surface part which projects from the rear part of the main housing 11a, as can be seen from figure 1. The outer surface of the operating button 13 further comprises an annular cylindrical part which is adjacent to the cone part shown in figure 1 and which extends into the rear part of the main housing 11 a. The annular cylindrical portion of the outer surface of the operating button 13 is engaged with the inner surface of the main housing 11 a. The surface of the cylindrical portion engaging the main housing 11a can be polished and in particular highly polished. Correspondingly, a portion of the inner surface of the main housing 11 a on the corresponding end part of the main housing 11a which faces the cylindrical portion can also be polished. By polishing surface portions of the main housing 11 a and/or the operating button 13, a low friction coefficient of the corresponding surfaces is achieved. Therefore, the operating button 13 can be actuated easily. Also, the abrasion on parts of the main housing 11 a and the operating button 13 is minimized.

According to an example of the first embodiment of the delivery device 10 shown in figure 1, the housing cap 12 and the operating button 13 could be made of different ceramic materials, wherein the different ceramic materials are selected in accordance with special needs or requirements of the medical delivery device 10. Since the main housing 11a contains the inner mechanic parts including the drive mechanism, the main housing 11 a made of ceramic material is formed as durable and stable for reliably supporting the components of the inner mechanic parts which are in engagement with parts of the main housing 11a.

By forming the main housing 11 a from a ceramic material which is different from the ceramic material of the housing cap 12 and of the operating button 13, the ceramic materials can be selected such that the difference of the two materials can be realized by a user and thereby an improvement of the outer visual impression given by the insulin pen 10 can be achieved. However, as an alternative, the ceramic material of the main housing 11a can additionally be coated with a further ceramic material, for example a layer of the first ceramic material, in order to achieve a more uniformly outer appearance, wherein in the same time the technical effects that are provided because of the use of the first ceramic material for the inner part of the main housing 11 a is also provided at the same time.

As can be seen from figure 1, the main housing 11a comprises a window 14 through which information of the actual dose setting is provided to the user. The main housing 11a can be formed with a frame element disposed on the main housing 11a such that it surrounds the window 14 and thereby serves for generally improving the visual impression of the delivery device 10 and also functionally supporting the user in drawing his attention to the information which can be seen through the window 14.

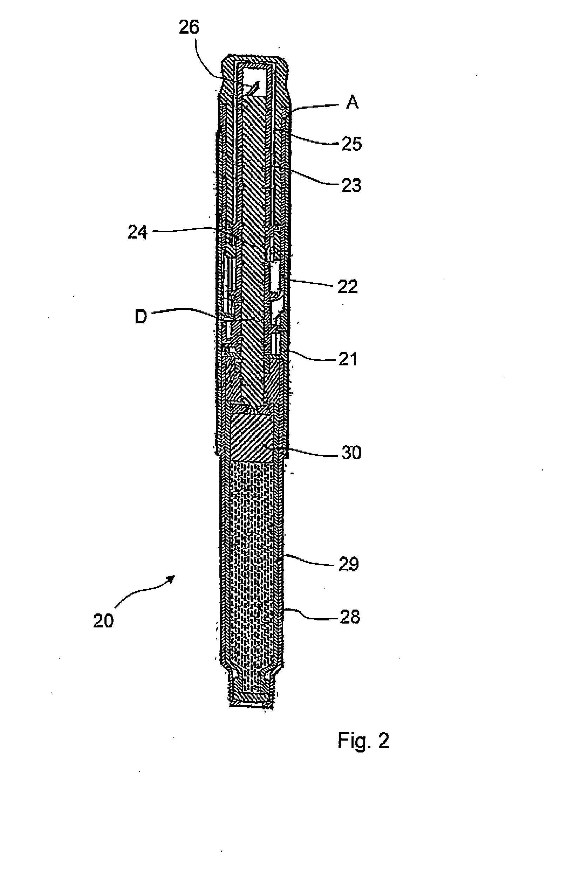

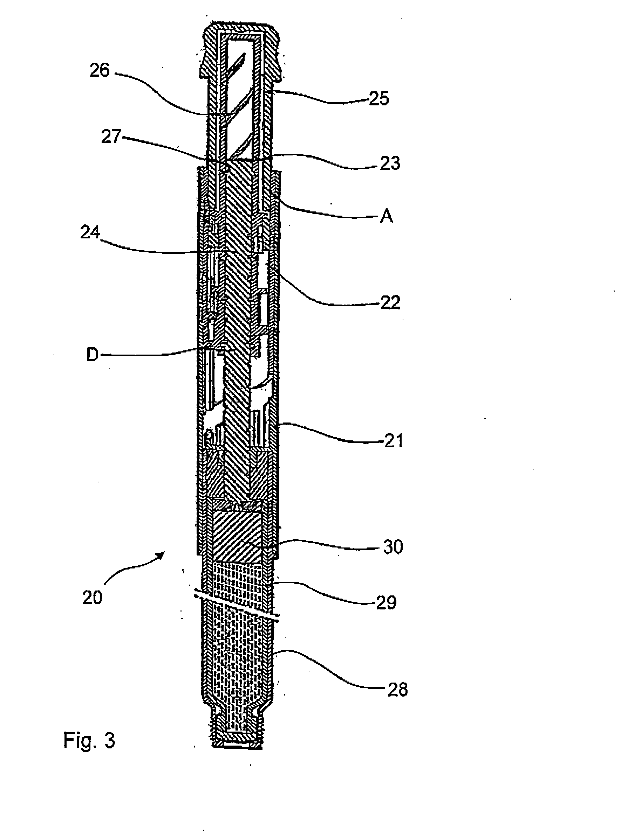

Referring to figures 2 and 3, a sectional view of a medical delivery device 20 according to a second embodiment of the invention is shown. Like the first embodiment, the medical delivery device 20 has also the shape of a pen.

The pen 20 comprises a housing with a main housing 21 and a front housing part 28. The front housing part 28 is designed for receiving a medicament cartridge 29. In the medicament cartridge 29 a piston 30 is movably disposed which in figure 2 is positioned at the provided on a rear end of the medicament cartridge 29. In the pen 20, the piston 30 is to be moved in a forward direction in order to expel a medicament from the cartridge 29.

The main housing 21 contains a drive mechanism D and the operating device A, which both are disposed in an inner space of the main housing 21. In particular, the drive mechanism D comprises a piston rod 24 on a forward end thereof the piston 30 is attached and a drive sleeve 23 in the main housing 21. Further, the operating device A comprises an inner housing sleeve 22 and a dosing sleeve 25 disposed at the rear end of the main housing 21, wherein the dosing sleeve 25 projects from a rear part of the main housing 21 for handling purposes. The pen can be manually operated by longitudinally moving the dosing sleeve 25 with regard to the main housing 21. When the user intends to set a desired dose of a medicament to be expelled during a injection, the dosing sleeve 25 is moved from a initial position in the proximal direction until a dosing position of the dosing sleeve 25 is reached that corresponds with the desired dose of medicament (cf. Figure 3). For expelling the selected amount of medicament, the dosing sleeve 25 is moved in the distal direction to its initial position. The dosing sleeve 25 is mounted into an inner housing sleeve 22 of the operating device 25. The inner housing sleeve 22 supports the dosing sleeve 25 and can serve as a thrust bearing means for the dosing sleeve 25.

Further the drive sleeve 23 of the drive mechanism D is functionally coupled to the dosing sleeve 25. In this embodiment, the drive sleeve 23 comprises an inner thread 26 and is in threaded engagement 27 with the piston rod 24 of the drive mechanism D for moving the piston rod 24. When the dosing sleeve 25 is moved in distal direction into the main housing 21, the drive sleeve 23 functions as coupling between the dosing sleeve 25 and the piston rod 24 in order to couple the movement of the dosing sleeve with a corresponding movement of the piston rod 24 and of the piston 30, wherein the distance covered by the piston 30 corresponds with the initially selected dose setting position of the dosing sleeve 25.

According to the invention, the main housing 21, the inner housing sleeve 22, the drive sleeve 25, the piston rod 24, the dosing sleeve 25, the front housing part 28 or other parts of the medicament pen 30 can be made of a ceramic material.
Further, in particular the drive sleeve 25 can be made of a ceramic material. According to a preferred embodiment, the ceramic of the drive sleeve 23 is polished, in particular highly polished, in order to obtain a low friction coefficient. As already mentioned above, all other parts of the pen 20 which need to have a low friction coefficient can be made of ceramic, for example the inner housing shell 22. Thereby, the functionality and reliability and easy running characteristic of the medicament pen 20 are improved.

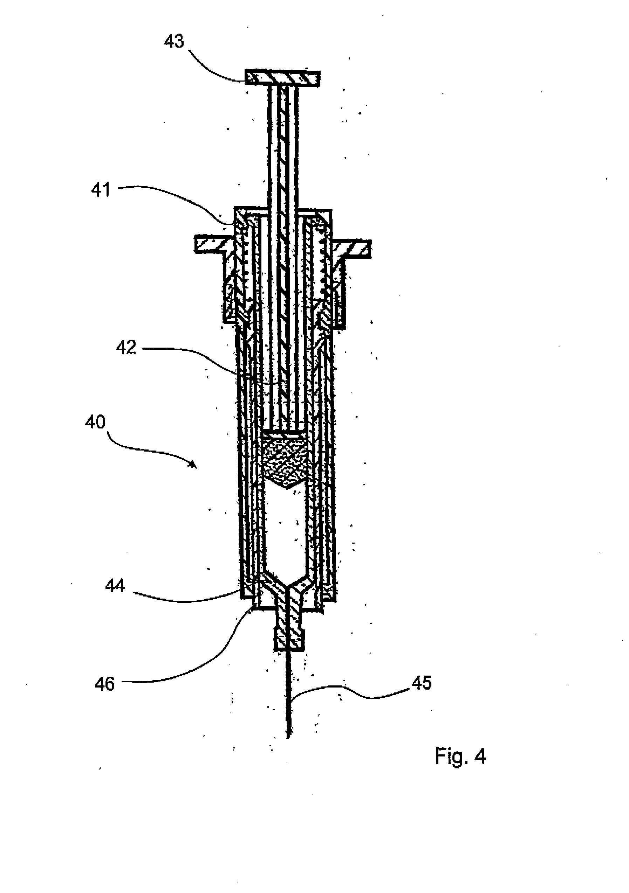

Referring to figure 4, a third embodiment of the invention is described. The medical delivery device 40 is a syringe containing a medicament ampoule 44. The medicament ampoule 44 is arranged in an inner housing part which functions as a cartridge holder and which is disposed in a outer housing part 41. The outer housing part 41 can be made of a ceramic material. As to an example of the invention, the inner housing part 46, for economical reasons, is made of a plastic material.

According to another example, the inner housing part 46 is made of a ceramic material. In a further embodiment, the inner housing part is made of ceramic material, wherein the outer housing part is made of a plastic material but is coated with a ceramic material. In such a embodiment, the total weight of the syringe 40 can be reduced in comparison to a syringe with a outer housing part 41 of massive ceramic.

The syringe 40 shown in Figure 4 further comprises a piston rod 42 as part of a drive mechanism D. The piston rod 42 can be made of a ceramic material, in particular, which can be different to the ceramic material of the outer housing part 41. On the proximal end of the piston rod 42 a handle 43 as part of an operating device is provided. The handle 43 can be made of ceramic material or can be coated with a ceramic material. According to the embodiment shown in Figure 4, the handle is formed as one piece with the piston rod 42.

The invention is not limited to devices shown in Figs. 1 to 4. In particular, the invention is not limited to medical devices having the form of a pen or a syringe as shown in Figure 4. For example, the invention can also be implemented in a disposable syringe or in any other form of portable medicine delivery devices or in not-portable medicine delivery devices.

## Claims

1. A medical delivery device (10, 20, 40) comprising:
a housing (11, 11a, 21, 28, 41, 46),
a drive mechanism (A) disposed within the housing (11, 11a, 21, 28, 41, 46) for expelling a medicament from a medicament reservoir,
an operating device (D) functionally coupled to the drive mechanism (A) for controlling the drive mechanism (A) when operated by a user,
wherein the housing (11, 21, 28, 41, 46) is formed by a shell made of a ceramic material.

2. Housing cap (12) for a medical delivery device, wherein the housing cap is formed by a shell made of ceramic material.

3. Medical delivery device (10, 20, 40) or housing cap (12) according to claims 1 or 2, wherein the shell of the housing (11, 21, 28, 41, 46) or the housing cap (12) comprises an outer surface that is at least partially polished.

4. Medical delivery device (10, 20, 40) or housing cap (12) according to any of the preceding claims, wherein the outer surface of the housing (11, 21, 28, 41, 46) or the housing cap (12) is at least partly coated with a layer of plastic material or a layer of metal or an additional layer of a ceramic material, wherein the material of the additional ceramic layer is different from the material of the shell.

5. Medical delivery device (10, 20, 40) or housing cap (12) according to any of the preceding claims, wherein the housing (11, 21, 28, 41, 46) comprises at least one polished surface which is provided on an area of the housing (11, 21, 28, 41, 46) which is in friction contact with parts of the drive mechanism (A) and/or with parts of the operation mechanism.

6. Medical delivery device (10, 20, 40) according to any of the preceding claims, comprising a cap (12) removable attachable to the housing (11, 21, 28, 41, 46) for protecting a proximal end of the housing (11, 21, 28, 41, 46) according to any of the preceding claims.

7. Medical delivery device (10, 20, 40) having a drive mechanism (A), wherein the drive mechanism (A) comprises at least one movable component which is rotatable or axially movable relative to the housing (11, 21, 28, 41, 46) and which is made of a ceramic material.

8. Medical delivery device (10, 20, 40) according to claim 7, wherein the movable component is a piston rod and/or a drive sleeve made of or coated with ceramic material.

9. Medical delivery device (10, 20, 40) according to claim 8, wherein an inner surface and/or an outer surface of the drive sleeve is co-acting with a piston rod that is made of ceramic material or at least partly coated with ceramic material.

10. Medical delivery device (10) according to any of the preceding claims 7 to 9, wherein the drive mechanism (A) comprises a dosing sleeve which is formed of ceramic material or coated with a ceramic material.

11. Medical delivery device (10) according to any of the preceding claims, wherein the drive mechanism (A) and/or the operating device (D) comprise
at least one first component that is moving relative to the housing (11, 21, 28, 41, 46) when the drive mechanism (A) is actuated and
at least one second component which is stationary relative to the housing (11, 21, 28, 41, 46) when the drive mechanism (A) is actuated,
wherein at least one second component is made of ceramic material or coated with ceramic material.

12. Medical delivery device (10, 20, 40) according to any of the preceding claims, wherein components made of ceramic material are formed in a ceramic injection moulding (CIM) process.

13. Method of forming a medical delivery device, wherein ceramic material is used for a shell of a housing (11, 21, 28, 41, 46) or of a cap (12) of the medical delivery device and/or for inner mechanical parts of the medical delivery device.

14. Method according to claim 13, wherein the ceramic parts are formed in a ceramic injection moulding (CIM) process and/or in a milling or cutting process.
